# EUROPEAN PATENT SPECIFICATION

(11) **EP 3 777 829 B1**
(45) Date of publication and mention of the grant of the patent: **29.05.2024**
(21) Application number: 20000273.1
(22) Date of filing: 05.08.2020
(51) Int. Cl.: A61K 9/00, A61K 9/08, A61K 31/135, A61K 47/12, A61K 47/24, A61P 25/24

(54) **ORAL PHARMACEUTICAL SOLUTIONS COMPRISING NORTRIPTYLINE HYDROCHLORIDE**
ORALE PHARMAZEUTISCHE LÖSUNGEN MIT NORTRIPTYLINHYDROCHLORID
SOLUTIONS PHARMACEUTIQUES ORALES COMPRENANT DU CHLORHYDRATE DE NORTRIPTYLINE

(30) Priority: 07.06.2019 GR 20190100252
(43) Date of publication of application: 17.02.2021
(73) Proprietor: Lamda Laboratories S.A., 19441 Koropi Attikis (Karellas) (GR)
(72) Inventor: Karatzas, Aggelos, 15235 Vrilissia (GR); Stappa, Argyro, 15234 Chalandri (GR); Stroumpou, Amalia, 15342 Agia Paraskevi (GR); Apostolou, Konstantinos, 15344 Gerakas (GR)

(56) References cited:
- WO-A1-98/05312
- WO-A1-2006/093493
- WO-A1-2015/144255
- US-A- 4 593 031
- Colonis: "SUMMARY OF PRODUCT CHARACTERISTICS Nortriptyline Colonis 10mg/5ml Oral Solution", , 4 June 2019 (2019-06-04), XP055763660, Retrieved from the Internet: URL:https://mhraproducts4853.blob.core.win dows.net/docs/dd0db68d39090b32bee2a3531348 bbaf37395607 [retrieved on 2021-01-11]
- Mallinckrodt Inc.: "Pamelor TM - nortriptyline HCl capsules USP nortriptyline HCl oral solution USP", , 31 October 2012 (2012-10-31), XP055763661, Retrieved from the Internet: URL:https://www.accessdata.fda.gov/drugsat fda_docs/label/2012/018012s029,018013s061l bl.pdf [retrieved on 2021-01-11]

## Description

### TECHNICAL FIELD

The present invention relates to stable pharmaceutical solutions suitable for oral administration comprising Nortriptyline hydrochloride.

### BACKGROUND OF THE INVENTION

Nortriptyline hydrochloride (Chemical formula I), the hydrochloride salt of Nortriptyline, is chemically named 10,11-Dihydro-N-methyl-5H-dibenzo [a,d]cycloheptene-Δ, γ-propylamine hydrochloride.

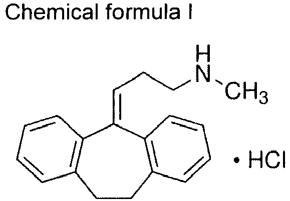

Nortriptyline, the N-demethylated active metabolite of amitriptyline, is a dibenzocycloheptene derivative tricyclic antidepressant. It exerts its antidepressant effects by inhibiting reuptake of norepinephrine and serotonin in CNS nerve terminals. It is characterised by a well-established safety and efficacy profile and is being broadly prescribed for the amelioration of depressive symptoms. It may also be used in children for the treatment of some cases of nocturnal enuresis.

Nortriptyline is only very slightly soluble in water, therefore the hydrochloric salt of Nortriptyline is generally employed to circumvent the limited solubility of the free base.

Currently, Nortriptyline is commercially available as film coated tablets and capsules in the strengths of 10 and 25 mg. It is also available in the United States as a 10 mg/5 ml oral solution. It is marketed under several trade names such as Aventyl, Noritren, Pamelor, Allegron and Nortrilen among others.

Oral solutions possess distinguishing features over solid dosage forms in terms of effective dose adjustment especially in the pediatric population, in which dose adaptation on a weight basis is applied, and improved adherence rates for individuals experiencing swallowing difficulties (for geriatric and psychiatric patients for instance). Therefore, the availability of stable, safe and efficacious Nortriptyline Oral Solutions that reproducibly deliver the therapeutic benefit declared in the label claim is of high clinical utility.

However, the commercially available Nortriptyline Oral Solutions contain ethanol, whose presence in oral adult medicines is discouraged for a number of health reasons including interactions with other medicines, diseases, effect on driving performances and issues with addiction (EMA/CHMP/507988/2013, Committee for Human Medicinal Products, 23 January 2014). Most importantly, ethanol is not a suitable excipient for medications intended for use in pediatric populations due to its toxicity.

Thus, while there have been attempts in the prior art to formulate stable child friendly compositions of Nortriptyline, there is still a strong need in the art for a composition or system that is also free from ethanol and has less irritation potential containing minimum quantities of non-friendly, particularly to the pediatric population, excipients such as sorbitol, polyethylene glycol, propylene glycol, glycerol, coloring agents, etc.

The present invention meets these needs.

### SUMMARY OF THE INVENTION

The present invention provides stable ethanol-free aqueous pharmaceutical solutions suitable for oral administration comprising Nortriptyline hydrochloride in association with a pharmaceutically acceptable liquid carrier.

According to the invention, said oral pharmaceutical solutions comprise Nortriptyline hydrochloride as active ingredient, sodium or potassium benzoate within the range from 0.1 mg/ml to 20 mg/ml, and purified water; wherein the pH of the oral solution is from 3.5 to 5.0.

The use of sodium or potassium benzoate at specific concentration ranges with simultaneous pH adjustment in the 3.5 to 5.0 range results in physicochemically stable aqueous oral solutions of Nortriptyline hydrochloride while at the same time providing sufficient antimicrobial activity.

The oral solutions of the present invention may also further comprise a pharmaceutically acceptable sweetening agent. The sweetening agent masks the inherent bitterness of the drug substance, thereby increasing medication compliance.

The aqueous oral pharmaceutical solutions of Nortriptyline hydrochloride according to the invention have the advantage of being physicochemically stable while providing sufficient antimicrobial activity without using ethanol, propylene glycol, polyethylene glycol, glycerol, sorbic acid as well as methyl p-hydroxybenzoate, propyl-p-hydroxybenzoate, ethyl-p-hydroxybenzoate or the sodium salts of these esters. This is particularly important when formulating oral solutions suitable for the pediatric population.

Additional advantages and features of the oral pharmaceutical solutions of the present invention will become apparent from the following description.

### DETAILED DESCRIPTION OF THE INVENTION

The present invention provides stable aqueous pharmaceutical solutions free of ethanol suitable for oral administration comprising Nortriptyline hydrochloride in association with a pharmaceutically acceptable liquid carrier.

As used throughout the present description and claims, the term «mg/ml», when referring to the active ingredient or inactive ingredients (excipients), means the milligrams of active or inactive ingredient per ml of oral solution.

As used throughout the present description and claims, the term "organic co-solvent" means any solvent which is miscible in a formulation in the amount desired and which, when added provides a formulation in which the medicament can be dissolved in therapeutically effective amounts. Examples of organic solvents frequently used in pharmaceutical formulations include polyethylene glycol (PEG) of various molecular weights (e.g., PEG 200, 300, 400, 540, 600, 900, 1000, 1450, 1540, 2000, 3000, 3350, 4000, 4600, 6000, 8000, 20,000, 35,000), propylene glycol, glycerol, sorbitol, ethyl alcohol, oils, ethyl oleate and dimethyl sulfoxide (DMSO).

As used throughout the present description and claims, a composition is considered free (or "essentially free") of ethanol or other organic co-solvent in case the concentration thereof in the composition does not exceed 1 % by weight, more in particular if the concentration thereof does not exceed 0.5 % per weight, even more in particular if the concentration thereof does not exceed 0.1 % by weight.

Shelf life after first use (In-use shelf life), as established by the document of European Medicines Agency "Note for Guidance on the in-use stability testing of human medicinal products" (CPMPQWP/2934/99), is a period of time during which a multi-dose product can be used whilst retaining quality within an accepted specification once the container is first opened.

The ability of antimicrobial preservatives to inhibit the growth of, or kill, microorganisms in pharmaceutical preparations is evaluated through Antimicrobial Efficacy Tests (AETs). As used throughout the present description, the term "AET" refers to the test for efficacy of antimicrobial preservation described in paragraph 5.1.3 of the European Pharmacopoeia (EP). The test consists of challenging the preparation, with a prescribed inoculum of suitable microorganisms, storing the inoculated preparation at ambient temperature, avoiding sunlight, withdrawing samples from the container at specified intervals of time and counting the microorganisms in the samples so removed. The preservative properties of the preparation are adequate if, under the conditions of the test, there is a significant fall or no increase, as appropriate, in the number of microorganisms in the inoculated preparation after 2, 7, 14 and 28 days. The EP criteria for evaluation of antimicrobial activity in oral preparations are given in the following table in terms of decrease in the number of viable microorganisms against the value obtained for the inoculum.

**Table 1 - EP, Criteria, for AET (limits in log reduction units)**

| | 2 days | 7 days | 14 days | 28 days |
|---|---|---|---|---|
| Bacteria | - | - | 3 | No increase |
| Moulds & Yeasts | - | - | 1 | No increase |

The formulation studies disclosed herein address the need to provide liquid formulations of nortriptyline hydrochloride that are free of ethanol and are devoid of non-friendly, particularly to the pediatric population, excipients such as organic co-solvents and parabens, while at the same time displaying excellent physicochemical stability during storage.

The non-existence of licensed ethanol-free liquid formulations of nortriptyline hydrochloride generated the need for an extensive compatibility study which was carried out for the identification of potential drug-excipient interactions and/or instability issues.

In the course of the same studies, the stability profile of nortriptyline hydrochloride as a function of pH was also studied under both ambient and accelerated stability conditions, in order to gain sound evidence of the optimum for stability pH region.

It was surprisingly found that the addition of organic solvents was not required for the production of a physicochemically stable composition of nortriptyline hydrochloride. The absence of organic solvents is highly desired in oral pharmaceutical formulations, especially formulations intended for use in the pediatric formulation, as their presence may raise additional safety concerns.

Even more surprisingly, compositions containing the active substance with sole addition of sodium benzoate exhibited physicochemical stability even under accelerated storage conditions.

The pH of the liquid carrier was identified as a critical parameter for the maintenance of the chemical and physical integrity of the product. At pH range between 3.5 and 5.0 the liquid carrier exhibits optimal thermal stability as evidenced by the very low impurity levels.

In one embodiment, the oral pharmaceutical solutions of the present invention comprise Nortriptyline hydrochloride as active ingredient, sodium or potassium benzoate within the range from 0.1 mg/ml to 20 mg/ml, and purified water; wherein the pH of the oral solution is from 3.5 to 5.0.

In another embodiment, the free of ethanol oral pharmaceutical solutions comprise nortriptyline hydrochloride as active ingredient, sodium or potassium benzoate within the range from 0.4 mg/ml to 4 mg/ml, and purified water; wherein the pH of the oral solution is from 3.5 to 5.0.

Preferably the oral pharmaceutical solutions according to the invention comprise nortriptyline hydrochloride at a concentration from 1 mg/ml to 50 mg/ml.

In a preferred embodiment, the pH of the oral solution is from 3.5 to 4.5.

The compositions of the present invention exhibit excellent physicochemical properties even when they do not contain organic solvents. Thus, preferably the aqueous pharmaceutical solutions of nortriptyline hydrochloride of the present invention do not contain organic solvents such as polyethylene glycol, glycerol, sorbitol, propylene glycol or combinations thereof, that may raise additional safety and toxicity issues.

A pharmaceutically acceptable sweetening agent may be added in the oral pharmaceutical solutions according to the invention. The sweetening agent masks the inherent bitterness of the drug substance, delivering additionally a pleasant aftertaste, thus increasing medication compliance. The sweetening agent may be a natural sweetening agent, a non-sugar based artificial sweetening agent, or a combination thereof.

The natural sweetening agent that may be used in the present invention comprise, amongst others, erythritol, xylitol, mannitol, maltitol, fructose, glucose, sucrose, maltose, or any combination thereof.

The non-sugar based artificial sweetening agents which may be used in the present invention comprise, amongst others, sucralose, saccharin sodium, saccharin, aspartame, acesulfame potassium or any combination thereof.

A suitable pharmaceutically acceptable sweetening agent according to the present invention is sucralose as described in the corresponding Ph. Eur. monograph.

The concentration of sucralose in the oral pharmaceutical solutions of the present invention is preferably within the range from 0.5 mg/ml to 10 mg/ml.

In a preferred embodiment, the concentration of sucralose is within the range from 0.5 mg/ml to 5 mg/ml. The additional benefit of using the concentration of sucralose within these levels is providing oral pharmaceutical solutions of nortriptyline hydrochloride with minimum quantities of the excipient, which is desired in compositions that are intended for use in the pediatric population, while at the same time at levels sufficient enough to retain its ability to mask the inherent bitterness of the drug substance.

The oral pharmaceutical solutions of the present invention are free of methyl p-hydroxybenzoate, propyl p-hydroxybenzoate, ethyl-p-hydroxybenzoate, the sodium salts of their esters, or combinations thereof. It is important that such compositions do not include parabens whose presence raises safety and toxicity issues.

The oral aqueous pharmaceutical solutions of nortriptyline hydrochloride, according to the invention may also optionally contain additional excipients commonly used in preparing oral liquid compositions, such as viscosity adjusting agents and flavouring agents.

The viscosity adjusting agents may be, for example, sodium carboxymethylcellulose, methylcellulose, hydroxypropylcellulose, polyvinylpyrrolidone, maltitol or any combination thereof.

Appropriate flavouring agents which may be used in the present invention comprise, amongst others, any of the many non-toxic natural or artificial flavouring agents known in the daily practice. In particular, the flavouring agents used may include one or more of a variety of natural or artificial fruit flavours. Alternatively, or in addition, the flavouring agents may include one or more^of natural or artificial vanilla, chocolate, and caramel flavourings, amongst others.

In a preferred embodiment, the oral pharmaceutical solutions of the present invention consist of 2.28 mg/ml Nortriptyline hydrochloride, 0.50 mg/ml sodium benzoate, 2.00 mg/ml sucralose and purified water, wherein the pH of the oral solution is from 3.5 to 4.5.

In another preferred embodiment, the oral pharmaceutical solutions of the present invention consist of 5.70 mg/ml nortriptyline hydrochloride, 0.50 mg/ml sodium benzoate, 2.00 mg/ml sucralose and purified water, wherein the pH of the oral solution is from 3.5 to 4.5.

The oral aqueous pharmaceutical solutions of nortriptyline hydrochloride of the present invention exhibit excellent in-use shelf life (i.e. shelf life after first use) when supplied in multi-dose containers. Specifically, they remain physicochemically stable and at the same time effectively preserved when stored at room temperature (20°C - 25°C) even when the containers are opened at least once a day for at least one month.

The compositions of the present invention may be prepared using methods well-known in the prior art. For example they may be prepared using the following process:
The prescribed quantities of active substance and excipients are weighed in suitable containers. The appropriate amount of purified water is added into the main compounding tank and then the specified amount of Nortriptyline hydrochloride is charged. The solution is stirred until complete dissolution. The amounts of sodium benzoate and sucralose are added sequentially to the content of step 1 while stirring. Complete dissolution of each excipient is ensured prior to the addition of the next in line. The pH is adjusted to 3.5 - 5.0 with the aid of Hydrochloric Acid solution 1.0N or Sodium Hydroxide 1.0N. Purified water is added to final volume under stirring. The final solution is filtered and decanted into suitable glass bottles.

### EXAMPLES

The following examples show the influence of the proposed carrier, according to the invention, on the stability of Nortriptyline hydrochloride and on the preservation of the oral solutions.

### EXAMPLE 1

Studies were conducted in order to identify the effect of several co-solvents to the stability of Nortriptyline hydrochloride oral solutions. Combinations of the active substance with constituents commonly used in oral solutions were evaluated for their possible stabilizing effect by being placed into stability chambers for a period of two months under extreme and accelerated conditions (i.e. 55°C & 40°C/ 75%RH). The levels of the related substances (impurities) produced were used as the responses of the study. In the course of the same study, the stability profile of nortriptyline hydrochloride as a function of pH was studied in order to gain sound evidence of the optimum for stability pH region. The concentration of related substances was estimated by HPLC.

**Table 2 - Excipient evaluation studies**

| | **Composition** | | | | | | |
|---|---|---|---|---|---|---|---|
| | **A1** | **A2** | **A3** | **A4** | **A5** | **A6** | **A7** |
| **Active ingredient** | mg/ml | | | | | | |
| Nortriptyline hydrochloride | 5.70 | 5.70 | 5.70 | 5.70 | 5.70 | 5.70 | 5.70 |

| **Excipients** | mg/ml | | | | | | |
|---|---|---|---|---|---|---|---|
| Ethanol 96% | - | - | 100.0 | - | - | - | - |
| Propylene glycol | - | - | - | 400.0 | - | - | - |
| Sorbitol 70% | - | - | - | - | 400.0 | - | - |
| Sodium benzoate | 1.0 | 1.0 | 1.0 | 1.0 | 1.0 | 1.0 | 1.0 |
| Sucralose | - | 3.0 | 3.0 | 3.0 | 3.0 | 3.0 | 3.0 |
| Hydrochloric acid 1 N | QS to pH 4.0 | | | | | QS to pH 6.0 | QS to pH 3.0 |
| Purified Water | QS to 1.0 ml | | | | | | |

**Table 3 - Stability data**

| **Test** | **Related Substances, T=0** | | | | | | |
|---|---|---|---|---|---|---|---|
| | **A1** | **A2** | **A3** | **A4** | **A5** | **A6** | **A7** |
| pH | 3.73 | 4.05 | 4.12 | 3.93 | 3.97 | 6.04 | 2.94 |
| Impurity A (%) | N.D. | N.D. | N.D. | N.D. | B.Q.L | ND | N.D. |
| Impurity D (%) | N.D. | N.D. | N.D. | N.D. | B.Q.L | N.D. | N.D. |
| Any other impurity (%) | B.R.L | B.R.L | B.Q.L | B.R.L | B.R.L | B.R.L | B.R.L |
| Total Impurities (%) | B.R.L | B.R.L | B.R.L | B.R.L | B.R.L | B.R.L | B.R.L |

| **Test** | **Related Substances, T=60 days at 55 °C** | | | | | | |
|---|---|---|---|---|---|---|---|
| | **A1** | **A2** | **A3** | **A4** | **A5** | **A6** | **A7** |
| pH | 4.22 | 4.13 | 4.02 | 4.14 | 3.90 | 6.15 | 2.89 |
| Impurity A (%) | N.D. | N.D. | B.Q.L | N.D. | N.D | 0.2 | 0.3 |
| Impurity D (%) | B.Q.L | B.Q.L | B.R.L | B.Q.L | B.Q.L | B.R.L | B.R.L |
| Any other impurity (%) | B.R.L. | B.Q.L. | B.Q.L. | B.R.L. | B.R.L. | 0.3 | 0.3 |
| | | | | | | RRT:0.17 | RRT:0.17 |
| | | | | | | 0.2 | 0.2 |
| | | | | | | RRT:0.28 | RRT:0.28 |
| | | | | | | 1.7 | 1.9 |
| | | | | | | RRT:0.41 | RRT:0.41 |
| | | | | | | 0.7 | 0.8 |
| | | | | | | RRT:0.49 | RRT:0.49 |
| Total Impurities (%) | B.R.L | B.R.L | B.R.L | B.R.L | B.R.L | 3.2 | 3.6 |

| **Test** | **Related Substances, T=60 days, 40°C ± 2°C/75% RH ± 5% RH** | | | | | | |
|---|---|---|---|---|---|---|---|
| | **A1** | **A2** | **A3** | **A4** | **A5** | **A6** | **A7** |
| pH | 4.10 | 3.96 | 4.13 | 3.91 | 4.08 | 6.12 | 3.05 |
| Impurity A (%) | N.D. | N.D. | N.D. | N.D. | N.D. | B.R.L | B.R.L |
| Impurity D (%) | N.D. | B.R.L | B.R.L | N.D. | N.D. | B.R.L | B.R.L |
| Any unknown impurity (%) | B.R.L. | B.R.L. | B.R.L. | B.R.L. | B.R.L. | 0.2 | 0.2 |
| | | | | | | RRT:0.41 | RRT:0.41 |
| | | | | | | 0.1 | 0.3 |
| | | | | | | RRT:0.50 | RRT:0.50 |
| Total Impurities (%) | B.R.L | B.R.L | B.R.L | B.R.L | B.R.L | 0.4 | 0.5 |

| | | | | | | | |
|---|---|---|---|---|---|---|---|
| B.R.L: Below Reporting Limit (0.1%), B.Q.L: Below quantitation limit, N.D: Not detected, RRT: Relative retention time | | | | | | | |

The inventors surprisingly found that the addition of ethanol (100mg/ml), propylene glycol (400 mg/ml) or liquid sorbitol 70% (400 mg/ml) was not required for the production of a physicochemically stable composition of Nortriptyline hydrochloride. Therefore, these excipients were excluded from further development.

As per the impact of pH on the quality performance of the mixtures, a kind of pH-stability dependency was evidenced. The optimal behaviours recorded were seen for the pH range around 4, while pH values around 3 or around 6 gave rise to unknown degradants whose kinetics were thermally catalysed.

### EXAMPLE 2 Study for the standardisation of preservative levels

Further optimization of the compositions was undertaken based on the initial formulation results. The preservation activity of sodium benzoate is elicited in acidic environments, therefore the acidity was initially fixed at pH 3.5 - 4.5, a value which is within the optimal pH range.

Sucralose may be added for the organoleptic appeal of the product, owing to its chemical inertness and its high sweetness intensity free of metallic aftertaste.

Consistent with the compendial (Ph. Eur.) requirements, the adequacy of the preservative agent of sodium benzoate in the optimized formulations was determined by means of preservative efficacy testing (PET). Furthermore, the oral solutions of nortriptyline are under considerable risk of contamination given their multidose nature, therefore the optimized compositions were also tested under conditions simulating in-use handling.

A series of trials were subjected to this challenge testing to identify the minimum quantity needed to deliver a product that maintains its microbiological integrity during its shelf life. The following compositions with a nortriptyline hydrochloride content of 5.70 mg/ml, sucralose content of 2.0 mg/ml at a pH value 3.5 - 4.5 and varying amounts of the preservative sodium benzoate were assessed: T1 (1.0 mg/ml), T2 (0.8 mg/ml), T3 (0.5 mg/ml) and T4 (0.45 mg/ml). Composition T4 was also tested under in-use stability.

The above solutions were prepared through a manufacturing process, where the prescribed quantities of active substance and excipients are weighed in suitable containers. The appropriate amount of purified water was added into the main compounding tank and then the specified amount of nortriptyline hydrochloride was charged. The solution was stirred until complete dissolution. The amounts of sodium benzoate and sucralose were added sequentially to the content of step 1 while stirring. Complete dissolution of each excipient is ensured prior to the addition of the next in line. The pH was adjusted to 3.5 - 4.5 with the aid of hydrochloric acid solution 1.0 N or sodium hydroxide solution 1.0 N. The solution goes to final volume with the addition of purified water, passed through a 40µm polypropylene cartridge filter and the solution was decanted into suitable glass bottles.

For the purposes of the in-use simulation, volumes of 1 ml were retrieved every day up to 30 days using the designated measuring syringe. The remaining volume was subjected to the relevant control.

By applying the pour plate method, as described in the relevant chapter of European Pharmacopoeia, the following results (i.e. colonies) were recorded at the time intervals specified in Table 4 below.

**Table 4 - Preservative efficacy testing results**

| **Preservative efficacy test of trial T1 (Sodium Benzoate 1.0 mg/ml)** | | | | | | |
|---|---|---|---|---|---|---|
| **Parameter** | **Inoculation** | **0 Time** | **2^{nd} Day** | **7^{th} Day** | **14^{th} Day** | **28^{th} Day** |
| P. aeruginosa | 8.0E+05 | 7.2E+05 | <100 | <10 | <10 | <10 |
| S. aureus | 6.1E+05 | 4.9E+05 | <100 | <10 | <10 | <10 |
| E.coli | 6.9E+05 | 6.2E+05 | <100 | <10 | <10 | <10 |
| C. albicans | 2.4E+05 | 2.4E+05 | <100 | <10 | <10 | <10 |
| A. brasiliensis | 4.5E+04 | 4.9E+04 | <100 | <10 | <10 | <10 |

| **Preservative efficacy test of trial T2 (Sodium Benzoate 0.8 mg/ml)** | | | | | | |
|---|---|---|---|---|---|---|
| **Parameter** | **Inoculation** | **0 Time** | **2^{nd} Day** | **7^{th} Day** | **14^{th} Day** | **28^{th} Day** |
| P. aeruginosa | 8.0E+05 | 7.1E+05 | <100 | <10 | <10 | <10 |
| S. aureus | 9.0E+05 | 9.5E+05 | <100 | <10 | <10 | <10 |
| E. coli | 9.0E+05 | 7.8E+05 | <100 | <10 | <10 | <10 |
| C. albicans | 2.5E+05 | 2.9E+05 | <100 | <10 | <10 | <10 |
| A. brasiliensis | 5.1E+04 | 3.3E+04 | <100 | <10 | <10 | <10 |

| **Preservative efficacy test of trial T3 (Sodium Benzoate 0.5 mg/ml)** | | | | | | |
|---|---|---|---|---|---|---|
| **Parameter** | **Inoculation** | **0 Time** | **2^{nd} Day** | **7^{th} Day** | **14^{th} Day** | **28^{th} Day** |
| P. aeruginosa | 8.0E+05 | 7.3E+05 | <100 | <10 | <10 | <10 |
| S. aureus | 9.0E+05 | 8.6E+05 | <100 | <10 | <10 | <10 |
| E. coli | 9.0E+05 | 7.4E+05 | <100 | <10 | <10 | <10 |
| C. albicans | 2.5E+05 | 3.2E+05 | <100 | <10 | <10 | <10 |
| A. brasiliensis | 5.1E+04 | 5.4E+04 | <100 | <10 | <10 | <10 |

| **Preservative efficacy test of trial T4 (Sodium Benzoate 0.45 mg/ml)** | | | | | | |
|---|---|---|---|---|---|---|
| **Parameter** | **Inoculation** | **0 Time** | **2^{nd} Day** | **7^{th} Day** | **14^{th} Day** | **28^{th} Day** |
| P. aeruginosa | 3.9E+05 | 2.6E+05 | <100 | <10 | <10 | <10 |
| S. aureus | 4.5E+05 | 3.8E+05 | <100 | <10 | <10 | <10 |
| E. coli | 5.3E+05 | 4.8E+05 | <100 | <10 | <10 | <10 |
| C. albicans | 2.9E+05 | 2.9E+05 | <100 | <10 | <10 | <10 |
| A. brasiliensis | 4.6E+05 | 3.7E+05 | 1.3E+03 | 2.4E+02 | 5.5E+01 | 4.5E+01 |

| **Preservative efficacy test of trial T4 (In-use testing)** | | | | | | |
|---|---|---|---|---|---|---|
| **Parameter** | **Inoculation** | **0 Time** | **2^{nd} Day** | **7^{th} Day** | **14^{th} Day** | **28^{th} Day** |
| P. aeruginosa | 4.9E+05 | 4.9E+05 | <100 | <10 | <10 | <10 |
| S. aureus | 4.4E+05 | 4.1E+05 | <100 | <10 | <10 | <10 |
| E. coli | 8.9E+05 | 8.4E+05 | <100 | <10 | <10 | <10 |
| C. albicans | 3.5E+05 | 4.0E+05 | <100 | <10 | <10 | <10 |
| A. brasiliensis | 7.8E+05 | 7.3E+05 | 2.3E+05 | 7.8E+04 | 1.0E+03 | 8.6E+01 |

As shown in Table 4, the compositions tested exhibit considerable resistance to the tested microorganisms, even after in-use handling.

Overall, assessing the entire body of data in conjunction with all the involved risk factors, a concentration of sodium benzoate as low as 0.5 mg/ml is deemed reasonable, providing the necessary margin of safety when loss of preservative efficacy and/or content occur over time and under use.

### EXAMPLE 3

Table 5 shows preferred compositions according to the present invention.

**Table 5 - Preferred compositions**

| | **Composition E1** | **Composition E2** | **Composition E3** |
|---|---|---|---|
| **Active ingredient** | mg/ml | | |
| Nortriptyline hydrochloride | 2.28 | 5.70 | 5.70 |

| **Excipients** | mg/ml | | |
|---|---|---|---|
| Sodium benzoate | 0.5 | 0.5 | 1.0 |
| Sucralose | 2.0 | 2.0 | 3.0 |
| Hydrochloric acid 1 N solution | QS to pH 3.5 - 4.5 | QS to pH 3.5 - 4.5 | QS to pH 3.5 - 4.5 |
| Purified Water | QS to 1.0 ml | QS to 1.0 ml | QS to 1.0 ml |

The effectiveness of the above standardised formulations was further demonstrated within the framework of a formal ICH stability program, as shown in Table 5. Quantification of sodium benzoate, Nortriptyline hydrochloride and impurity content in the solutions was performed by HPLC.

**Table 6 - Stability data for compositions E1 to E3**

| **Test** | **Specifications** | **T = 0** | | |
|---|---|---|---|---|
| | | **Composition E1** | **Composition E2** | **Composition E3** |
| pH | 3.5 - 4.5 | 4.0 | 4.1 | 4.0 |
| Assay | 95.0 -105.0% | 100.1% | 102.5% | 99.5% |
| Preservative content | 90.0 -110.0% | 100.7% | 99.8% | 98.9% |
| Impurity A (%) | NMT 0.25% | N.D. | N.D. | N.D. |
| Impurity D (%) | NMT 0.2% | B.R.L. | B.R.L. | B.R.L. |
| Any other impurity (%) | NMT 0.2% | B.R.L. | B.R.L. | B.R.L. |
| Total Impurities (%) | NMT 1.0 % | B.R.L. | B.R.L. | B.R.L. |

| **Test** | **Specifications** | **T = 6 months, 25°C / 60%RH** | | |
|---|---|---|---|---|
| | | **Composition E1** | **Composition E2** | **Composition E3** |
| pH | 3.5 - 4.5 | 4.0 | 4.1 | 4.1 |
| Assay | 95.0 -105.0% | 102.8% | 101.2% | 100.4% |
| Preservative content | 90.0 -110.0% | 100.1% | 99.2% | 99.5% |
| Impurity A (%) | NMT 0.25% | N.D. | B.D.L. | B.D.L. |
| Impurity D (%) | NMT 0.2% | B.R.L. | N.D. | N.D. |
| Any other impurity (%) | NMT 0.2% | B.R.L. | B.R.L. | B.R.L. |
| Total Impurities (%) | NMT 1.0% | B.R.L. | B.R.L. | B.R.L. |

| **Test** | **Specifications** | **T** = **6 months, 40°C / 75%RH** | | |
|---|---|---|---|---|
| | | **Composition E1** | **Composition E2** | **Composition E3** |
| pH | 3.5-45 | 4.0 | 4.1 | 3.9 |
| Assay | 95.0 -105.0% | 103.4% | 101.0% | 99.2% |
| Preservative content | 90.0 -110.0% | 100.0% | 99.3% | 98.5% |
| Impurity A (%) | NMT 0.25% | N.D. | B.D.L. | B.D.L. |
| Impurity D (%) | NMT 0.2% | B.R.L | N.D. | N.D. |
| Any other impurity (%) | NMT 0.2% | B.R.L. | B.R.L. | B.R.L. |
| Total Impurities (%) | NMT 1.0 % | B.R.L. | B.R.L. | B.R.L. |

| | | | | |
|---|---|---|---|---|
| B.R.L: Below Reporting Limit (0.1%), B.D.L: Below detection limit, N.D: Not detected | | | | |

The results show excellent in-use shelf life. Specifically, the compositions remain physicochemically stable and at the same time effectively preserved even when stored for six months under stringent thermal and humidity conditions (40°C / 75%RH).

## Claims

1. Oral pharmaceutical solution free of ethanol comprising Nortriptyline hydrochloride, sodium or potassium benzoate within the range from 0.1 mg/ml to 20 mg/ml, and purified water; wherein the pH of the oral solution is from 3.5 to 5.0.

2. The oral pharmaceutical solution according to claim 1, wherein the sodium or potassium benzoate is within the range from 0.4 mg/ml to 4 mg/ml.

3. The oral pharmaceutical solution according to claim 1 or 2, wherein the concentration of Nortriptyline hydrochloride is from 1 mg/ml to 50 mg/ml.

4. The oral pharmaceutical solution according to any one of claims 1 to 3, wherein the pH of the oral solution is from 3.5 to 4.5.

5. The oral pharmaceutical solution according to any one of claims 1 to 4, which is free of organic co-solvents selected from the group comprising polyethylene glycol, glycerol, sorbitol, propylene glycol and mixtures hereof.

6. The oral pharmaceutical solution according to any one of claims 1 to 5, which further comprises a pharmaceutically acceptable sweetening agent.

7. The oral pharmaceutical solution according to claim 6, wherein the pharmaceutically acceptable sweetening agent is sucralose at a concentration within the range from 0.5 mg/ml to 10 mg/ml.

8. The oral pharmaceutical solution according to claim 7, wherein the concentration of sucralose is within the range from 0.5 mg/ml to 5 mg/ml.

9. The oral pharmaceutical solution according to any one of claims 1 to 8, which is free of methyl p-hydroxybenzoate, propyl p-hydroxybenzoate, ethyl-p-hydroxybenzoate, the sodium salts of their esters, or combinations thereof.

10. The oral pharmaceutical solution according to claim 1, which consists of
2.28 mg/ml Nortriptyline hydrochloride
0.50 mg/ml Sodium Benzoate
2.00 mg/ml Sucralose
and purified water, wherein the pH of the oral solution is from 3.5 to 4.5.

11. The oral pharmaceutical solution according to claim 1, which consists of
5.70 mg/ml Nortriptyline hydrochloride
0.50 mg/ml Sodium Benzoate
2.00 mg/ml Sucralose
and purified water, wherein the pH of the oral solution is from 3.5 to 4.5.

## Patentansprüche

1. Orale pharmazeutische Lösung ohne Ethanol bestehend aus Nortriptylin Hydrochlorid, Natrium- oder Kaliumbenzoat in einer Konzentration zwischen 0,1 mg/ml und 20 mg/ml und gereinigtes Wasser, wobei der pH-Wert der oralen Lösung zwischen 3,5 und 5,0 liegt.

2. Orale pharmazeutische Lösung gemäß Anspruch 1, wobei die Konzentration von Natrium- oder Kaliumbenzoat zwischen 0,4 mg/ml und 4 mg/ml liegt.

3. Orale pharmazeutische Lösung gemäß Anspruch 1 oder 2, wobei die Konzentration von Nortriptylin zwischen 1 mg/ml und 50 mg/ml liegt.

4. Orale pharmazeutische Lösung gemäß einem der Ansprüche 1 bis 3, wobei der pH-Wert der oralen Lösung zwischen 3,5 und 4,5 liegt.

5. Orale pharmazeutische Lösung gemäß einem der Ansprüche 1 bis 4, die frei von organischen Co-Lösungsmitteln, ausgewählt aus einer Gruppe, die Polyethylenglykol, Glycerol, Sorbitol, Propylenglykol und verschiedenen Mischungen enthält.

6. Orale pharmazeutische Lösung gemäß einem der Ansprüche 1 bis 5, die zusätzlich einen pharmazeutisch zugelassenen Süßstoff enthält.

7. Orale pharmazeutische Lösung gemäß Anspruch 6, wobei der pharmazeutisch zugelassene Süßstoff Sucralose in einer Konzentration zwischen 0,5 mg/ml und 10 mg/ml ist.

8. Orale pharmazeutische Lösung gemäß Anspruch 7, wobei die Konzentration von Sucralose im Bereich von 0,5 mg/ml bis 5 mg/ml liegt.

9. Orale pharmazeutische Lösung gemäß einem der Ansprüche 1 bis 8, die frei von Methyl-p-hydroxybenzoat, Propyl-p-hydroxybenzoat, Ethyl-p-hydroxybenzoat, die Natriumsalze aus deren Ester oder Kombinationen davon ist.

10. Orale pharmazeutische Lösung gemäß Anspruch 1, die aus 2,28 mg/ml Nortriptylin Hydrochlorid 0,50 mg/ml Natriumbenzoat 2,00 mg/ml Sucralose und gereinigtem Wasser besteht, während der pH-Wert der oralen Lösung zwischen 3,5 und 4,5 liegt.

11. Orale pharmazeutische Lösung gemäß Anspruch 1, die aus 5,70 mg/ml Nortriptylin Hydrochlorid 0,50 mg/ml Natriumbenzoat 2,00 mg/ml Sucralose und gereinigtem Wasser besteht, während der pH-Wert der oralen Lösung zwischen 3,5 und 4,5 liegt.

## Revendications

1. Solution pharmaceutique buvable sans éthanol comprenant du chlorhydrate de nortriptyline, du benzoate de sodium ou de potassium dans une plage entre 0,1 mg/ml et 20 mg/ml, et de l'eau purifiée, le pH de la solution buvable étant de 3,5 à 5,0.

2. Solution pharmaceutique buvable selon la revendication 1, dans laquelle la concentration de benzoate de sodium ou de potassium se situe dans la plage de 0,4 mg/ml à 4 mg/ml.

3. Solution pharmaceutique buvable selon la revendication 1 ou 2, dans laquelle la concentration de chlorhydrate de nortriptyline se situe entre 1 mg/ml et 50 mg/ml.

4. Solution pharmaceutique buvable selon l'une quelconque des revendications 1 à 3, dans laquelle le pH de la solution orale se situe entre 3,5 et 4,5.

5. Solution pharmaceutique buvable selon l'une quelconque des revendications 1 à 4, exempte de co-solvants organiques choisis dans le groupe comprenant le polyéthylène glycol, le glycérol, le sorbitol, le propylène glycol et leurs mélanges.

6. Solution pharmaceutique buvable selon l'une quelconque des revendications 1 à 5, comprenant en outre un agent édulcorant pharmaceutiquement acceptable.

7. Solution pharmaceutique buvable selon la revendication 6, dans lequel l'agent édulcorant pharmaceutiquement acceptable est le sucralose, à une concentration comprise dans la plage allant de 0,5 mg/ml à 10 mg/ml.

8. Solution pharmaceutique buvable selon la revendication 7, dans laquelle la concentration de sucralose se situe dans la plage de 0,5 mg/ml à 5 mg/ml.

9. Solution pharmaceutique buvable selon l'une quelconque des revendications 1 à 8, exempte de p-hydroxybenzoate de méthyle, de p-hydroxybenzoate de propyle, de p-hydroxybenzoate d'éthyle, de sels de sodium de leurs esters, ou de combinaisons de ceux-ci.

10. Solution pharmaceutique buvable selon la revendication 1, consistant en 2,28 mg/ml de chlorhydrate de nortriptyline, 0,50 mg/ml de benzoate de sodium, 2,00 mg/ml de sucralose et d'eau purifiée, dans laquelle le pH de la solution buvable se situe entre 3,5 et 4,5.

11. Solution pharmaceutique buvable selon la revendication 1, consistant en 5,70 mg/ml de chlorhydrate de nortriptyline, 0,50 mg/ml de benzoate de sodium, 2,00 mg/ml de sucralose et d'eau purifiée, dans laquelle le pH de la solution buvable se situe entre 3,5 et 4,5.
